# EUROPEAN PATENT SPECIFICATION

(11) **EP 1 522 321 B1**
(45) Date of publication and mention of the grant of the patent: **29.04.2009**
(21) Application number: 04077724.5
(22) Date of filing: 06.10.2004
(51) Int. Cl.: A61L 12/08, A61L 12/14

(54) **Method for disinfecting and/or cleaning contact lenses, using a liquid aqueous composition comprising at least one constituent of the aloe vera plant**
Verfahren zur Desinfektion und/oder Reinigung von Kontaktlinsen unter Verwendung einer wässrigen Lösung enthaltend wenigstens einen Bestandteil der Aloe Vera Pflanze
Méthode pour désinfecter et/ou nettoyer des verres de contact, en utilisant une composition aqueuse liquide comportant au moins un constituant de la plante d'aloès (aloe vera)

(30) Priority: 10.10.2003 NL 1024513
(43) Date of publication of application: 13.04.2005
(73) Proprietor: Jon Holding B.V., 5403 PD Uden (NL)
(72) Inventor: Oorthuizen, Rob Willem, 5403 TR Uden (NL); Oorthuizen, Wilhelmus Antonius Maria, 5403 PD Uden (NL)
(74) Representative: Valkonet, Rutger

(56) References cited:
- US-A- 4 401 582
- US-A- 6 013 259
- US-A- 6 162 393
- US-B1- 6 565 894
- PATENT ABSTRACTS OF JAPAN vol. 2000, no. 26, 1 July 2002 (2002-07-01) & JP 2001 264707 A (CHISSO CORP), 26 September 2001 (2001-09-26)

## Description

The present invention relates to a method for disinfecting and cleaning one or more contact lenses, wherein the contact lenses are brought into contact with a composition that comprises an active ingredient.

A method for disinfecting and/or cleaning contact lenses is known from European patent No. 0 648 131. Said method comprises the treatment of contact lenses with a so-called All-in-One composition for disinfecting, cleaning, storing, placing and wearing contact lenses. When such an All-in-One composition is used, the contact lenses are placed directly from the cleaning solution in the eyes. The active ingredient that is used according to the aforesaid European patent specification No. 0 648 131 is grapefruit seed extract.

The problem that is encountered when using the composition according to EP-0 648 131 is that the grapefruit seed extract, when used in high concentrations, may cause eye irritation and/or sensitivity, which seriously affects the degree of comfort and wearability of the lenses.

U.S. patent 6,162,393 relates to a contact lens solution, comprising the disinfectant compound, BDT (2-[2-(p-1,1,3,3-tetramethylbutylfenoxy)ethoxy]ethyl]ammoniumchloride, which can cause irratation of the eyes.

U.S. patent 6,565,894 relates to a ophthalmic solution comprising hydrogen pyrroxide and hystidine, which can cause irratation of the eyes.

U.S. patent 4,401,582 relates to a method for desinfecting contact lenses using a desinfectant composition comprising ascorbic acid, which can cause irratation of the eyes.

Japanse patent application 2001264707 relates to a desinfecting and preserving solution for contact lenses comprising polylysing and/or protamine and neutral or basic amino acids, which can cause irratation of the eyes.

The present invention has for its object to solve these eye irritation problems by providing a new method for cleaning and disinfecting contact lenses, which enhances the comfort and wearability of the lenses and which furthermore has a good disinfecting and cleaning effect.

The present invention provides a method for disinfecting and cleaning one or more contact lenses according to the preamble of claim 1, which is characterized in that the active ingredient is selected from the group consisting of natural Aloe Vera, synthetic Aloe Vera, aloin and one or more combinations thereof.

Aloe Vera is a very well-known natural product, which is frequently used in a large variety of medical and cosmetic products. The use of Aloe Vera for treating eye disorders is known, for example from US patents Nos. 6,013,259 and 4,788,007. US Patent No. 6,013,259 describes the use of an Aloe Vera-containing ophthalmic composition for treating eye disorders, such as dry eye syndrome, inflammations and ulcerations. The composition is applied to the eye in the form of eye drops, a gel or an ointment. Said document does not describe the use of Aloe Vera in a composition for disinfecting and cleaning contact lenses.

In addition to that, US patent No. 4,788,007 describes the use of an Aloe Vera-containing gel to protect against UV radiation; again no mention is made in said document of the use of Aloe Vera in a composition for disinfecting and cleaning contact lenses, however. Aloe Vera is thus considered to have a wide range of beneficial effects, such as an analgetic, a disinfecting and an anti-inflammatory effect. It furthermore stimulates the growth of new tissue, increases the level of immunity, it has a protecting and alleviating effect as regards UV radiation and does not exhibit any negative side-effects or toxicity.

In the present invention, one or more Aloe Vera constituents are used in a method for disinfecting and cleaning contact lenses, because of the disinfecting, cleaning and, in addition to that, preserving and comfort-enhancing effect of Aloe Vera. These properties of Aloe Vera ensure a good disinfection and cleaning result without having any adverse effect on the eye. It is surprising to find that contact lenses can be disinfected and cleaned in such a simple manner, and that can be placed in the eye directly after the contact lenses have been disinfected and cleaned, with the Aloe Vera having an additional comfort-increasing. It is also possible to store the contact lenses in the present composition, however, until they are worn at a later point in time. The present composition may furthermore be used in placing the contact lenses in the eye.

The composition as used in the present invention, to which the active ingredient according to the present invention is added, may be any composition that is known to those skilled in this field of the art for use in disinfecting and/or cleaning or storing contact lenses. Examples of such liquid aqueous compositions that can be used as a base for the present compositions comprise so-called All-in-One compositions, compositions for use in a two-stage cleaning process, such as a neutralisation fluid that is used after cleaning with hydrogen peroxide, an isotonic saline solution, a hypo-allergenic composition or one or more compositions that can be used in the overall care and use of contact lenses, although the invention is not limited to such compositions.

In addition to that, the present compositions and methods may be used with all types of contact lenses that are known to those skilled in this field of the art, such as hard lenses, soft plastic lenses and the like.

The present invention is preferably carried out with a composition in which the Aloe Vera constituents are present in the form of natural Aloe Vera. Such natural Aloe Vera is commercially available, for example in the form of a concentrate, a gel or a solution, and it is extracted from the Aloe plant by known methods. It is also possible to use synthetic variants of Aloe Vera in the present invention, however, as well as one or more constituents of natural or synthetic Aloe Vera, for example aloin, the most important active constituent of Aloe Vera.

A currently preferred concentration of Aloe Vera for use in the present method and composition ranges from 0.001 to about 10 wt.%. If a concentration of less than 0.001 wt.% is used, hardly any effect will be obtained, if at all. The maximum concentration that is currently preferred is a concentration of about 10 wt.%. Such a concentration will provide a suitable composition, which can be used in the present method without any undesirable side-effects. It will be understood, however, that also higher concentrations may be used.

An especially preferred concentration of Aloe Vera for use in the present invention is a concentration of about 3 - 10 wt.%, since experiments have shown that optimum results are obtained in this range.

Preferably, the composition comprises a liquid, aqueous medium, because such a medium provides a good compatibility with the eye.

In particular, the composition comprises an agent for adjusting the tonicity. In this way an isotonic composition can be obtained which is very compatible with the eye and which makes it possible to place the cleaned e.g. contact lens directly in the eye without the contact lens having to adapt to the eye fluid.

The pH-value of the composition that is used in the present invention preferably ranges from 4.5 to 8.5, because the use of compositions having a pH-value outside this range may lead to eye irritations.

In a further embodiment of the present method, a composition is used which comprises additives such as buffer agents, other cleaning agents, moisturisers, nutrients, viscosity-adjusting substances, care products, preservatives, antioxidants, UV-protection substances and the like. Said further additives may each be added to the present composition to impart desired or advantageous properties to the solution. Examples of such substances and any other additives are known to those skilled in the art.

Preferably, Aloe Vera is present in a concentration of 0.001 to about 10 the wt.%, in particular in a concentration of about 3 to about 10 wt.%, because it has been found that the best properties of the composition are obtained when these concentrations are used.

The composition to be used in the present method is preferably a liquid, aqueous composition, because such a composition is quite compatible with the eye fluid and with the eye.

Preferably, the composition comprises an agent for adjusting the tonicity so as to obtain an isotonic composition that is favourable to the eye.

For reasons of compatibility with the eye, the composition preferably has a pH-value in the 4.5-8.5 range.

In addition, the composition may comprise additives that can be selected from the group of additives as described above.

In a particularly preferred embodiment, the present invention comprises a composition consisting of:
(a) a physiological solution
(b) one or more buffer agents for stabilising the pH-value of the composition
(c) an active ingredient selected from the group consisting of natural Aloe Vera, synthetic Aloe Vera, aloin and one or more combinations thereof.
(d) the rest being made up of water.

This composition provides a disinfecting, cleaning and preserving effect and, in addition to that, it has an alleviating and comfort-enhancing effect on the eye.

The physiological solution as referred to under (a) is comparable to tear-water and has comparable physical and chemical properties. Such physiological solutions are known to those skilled in this field of the art.

The buffer agents as referred to under (b) comprise buffer agents that are commonly used in contact lens fluids; such buffer agents are known to those skilled in this field of the art. Examples of such buffer agents are: a phosphate buffer, a citrate buffer or a borate buffer and the like, or combinations thereof. Said buffer agents are used for stabilising the pH-value of the solution, as a result of which a stable solution is obtained which will retain its stability over time. The pH-value is preferably adjusted and stabilised at 4.5-8.5.

The constituent or constituents of Aloe Vera that are added as the active ingredient in step (c) are preferably added in a concentration of 0.001 to 10 wt.%; said constituents can be added in various forms, as will be apparent to those skilled in this field of the art. Examples thereof are: a concentrate, a solution, a suspension, a gel, powder and the like.

The composition is further supplemented with water in step (d). Other additives, such as the aforesaid additives, may be added, however, in order to obtain specific desired properties.

It will be apparent to those skilled in this field of the art that any alterations to the methods and compositions according to the present invention are possible without departing from the scope of the invention.

The present invention will now be explained in more detail by means of a few examples and comparative examples. The examples merely function to illustrate the invention, however, and do not limit the scope thereof.

### EXAMPLES

### Example 1. Preparation of an isotonic saline solution containing 6% Aloe Vera

Using one or more methods that are known to those skilled in this field of the art, a composition for disinfecting, cleaning, storing, placing and wearing contact lenses was prepared according to the following formulation:

| Constituent | Amount |
|---|---|
| Sodium chloride | 9 grammes |
| Aloe Vera concentrate (99.9%) | 60 grammes |
| Distilled water | 1 litre |

### Example 2. Use of an isotonic saline solution containing 6% Aloe Vera.

The fluid prepared according to Example 1 was subjected to a test carried out on 5 persons. Said persons used the present fluid for a period of 3 months for disinfecting, cleaning, storing, placing and wearing contact lenses. The assessment of said persons was as follows. All persons were allergic to the commonly used cleaning agents and contact lens fluids, experiencing irritation of the eyes and/or dry eyes. During the period that the present composition according to Example 1 was used, none of these phenomena occurred. All persons noticed an improvement as regards the irritations and/or dry eyes, and none of the persons experienced an allergic reaction, in contrast to their experiences with the use of an isotonic saline solution containing a different cleaning agent. The overall impression of the test subjects was that the present fluid provides greater comfort upon placing and wearing the contact lenses, without any irritations and/or dry eyes occurring.

The present composition can thus be used for disinfecting, cleaning, storing, placing and wearing contact lenses, and provides greater comfort and wearability than the solutions that are known so far, whilst it is furthermore suitable for people suffering from allergic reactions to other, known products.

## Claims

1. A method for disinfecting and cleaning one or more contact lenses, wherein the contact lenses are brought into contact with a composition that comprises an active ingredient, **characterized in that** the active ingredient is selected from the group consisting of natural Aloe Vera, synthetic Aloe Vera, aloïn and one or more combinations thereof.

2. A method according to claim 1, **characterized in that** the composition comprises the active ingredient in an amount of 0.001 to about 10 wt.%

3. A method according to claim 2, **characterized in that** the composition comprises Aloe Vera constituents in an amount of about 3 to about 10 wit.%.

4. A method according to any one or more of the preceding claims, **characterized in that** the composition comprises a liquid, aqueous medium.

5. A method according to any one or more of the preceding claims, **characterized in that** the composition comprises an agent for adjusting the tonicity.

6. A method according to any one or more of the preceding claims, **characterized in that** the composition has a pH-value in the 4.5-8.5 range.

7. A method according to any one or more of the preceding claims, **characterized in that** the composition furthermore comprises additives.

## Patentansprüche

1. Verfahren zum Desinfizieren und Reinigen von einer oder mehreren Kontaktlinsen, wobei die Kontaktlinsen in Kontakt mit einer Zusammensetzung gebracht werden, die einen aktiven Inhaltsstoff aufweist, **dadurch gekennzeichnet, dass** der aktive Inhaltsstoff aus einer Gruppe ausgewählt ist, die aus natürlicher Aloe Vera, synthetischer Aloe Vera, Aloin und aus einer oder mehreren Kombinationen davon besteht.

2. Verfahren gemäß Anspruch 1, **dadurch gekennzeichnet, dass** die Zusammensetzung den aktiven Inhaltsstoff in einer Menge von 0,001 bis etwa 10 Gewichts-% enthält.

3. Verfahren gemäß. Anspruch 2, **dadurch gekennzeichnet, dass** die Zusammensetzung Aloe Vera Bestandteile in einer Menge von etwa 3 bis etwa 10 Gewichts-% aufweist.

4. Verfahren gemäß einem oder mehreren der vorhergehenden Ansprüche, **dadurch gekennzeichnet, dass** die Zusammensetzung ein flüssiges, wässriges Medium aufweist.

5. Verfahren gemäß einem oder mehreren der vorhergehenden Ansprüche, **dadurch gekennzeichnet, dass** die Zusammensetzung ein Agens bzw. einen Stoff zum Einstellen der Tonizität aufweist.

6. Verfahren gemäß einem oder mehreren der vorhergehenden Ansprüche, **dadurch gekennzeichnet, dass** die Zusammensetzung einen pH-Wert im Bereich von 4,5 - 8,5 hat.

7. Verfahren gemäß einem oder mehreren der vorhergehenden Ansprüche, **dadurch gekennzeichnet, dass** die Zusammensetzung ferner Additive bzw. Zusatzstoffe aufweist.

## Revendications

1. Procédé de désinfection et de nettoyage d'une ou plusieurs lentilles de contact, les lentilles de contact étant mises en contact avec une composition qui comprend un ingrédient actif, **caractérisé en ce que** l'ingrédient actif est choisi dans le groupe comprenant de l'aloe vera naturel, de l'aloe vera synthétique, de l'aloïne et une ou plusieurs combinaisons de ceux-ci.

2. Procédé selon la revendication précédente, **caractérisé en ce que** la composition comprend l'ingrédient actif en une quantité de 0,001 à environ 10 % en poids.

3. Procédé selon la revendication 2, **caractérisé en ce que** la composition comprend des constituants d'aloe vera en une quantité d'environ 3 à environ 10 % en poids.

4. Procédé selon l'une ou plusieurs des revendications précédentes, **caractérisé en ce que** la composition comprend un milieu aqueux liquide.

5. Procédé selon l'une ou plusieurs des revendications précédentes, **caractérisé en ce que** la composition comprend un agent d'ajustement de tonicité.

6. Procédé selon l'une ou plusieurs des revendications précédentes, **caractérisé en ce que** la composition a un pH de l'ordre de 4,5 à 8,5.

7. Procédé selon l'une ou plusieurs des revendications précédentes, **caractérisé en ce que** la composition comprend en outre des additifs.
